# EUROPEAN PATENT APPLICATION

(11) **EP 0 659 438 A1**
(43) Date of publication of application: **28.06.1995**
(21) Application number: 93120789.8
(22) Date of filing: 23.12.1993
(51) Int. Cl.: A61K 47/48

(54) **Conjugates consisting of peptidic T cell antigens and cell binding groups, and their use for therapy**

(71) Applicant: BOEHRINGER MANNHEIM GMBH, D-68305 Mannheim (DE)
(72) Inventor: Mach, Jean-Pierre, Prof. Dr., CH-1010 Lausanne (CH); Healy, Frank, Dr., CH-1004 Lausanne (CH); Corradin, Giampietro, Dr., CH-Lausanne 26 (CH)

(57) **Abstract**

A conjugate comprising an essentially non-toxic peptidic T cell antigen, which is capable of forming a complex with MHC class I or II molecules, and a binding partner for a surface receptor on mammalian target cells, whereby
a) the binding partner has a high degree of selectivity for said target cells by binding to substances which are expressed on the surface of said cells,
b) the conjugate comprising the binding partner and the T cell antigen is internalized in said cells after binding of the binding partner to its surface receptor on said cells,
c) the T cell antigen is processed from the conjugate and expressed on the cell surface in the form of a complex with MHC molecules, whereby said complex is recognized by the T cell receptor, thereby inducing cytotoxicity of T cells for the aforesaid target cells,
is useful to induce specific cytotoxicity of T cells in the treatment of cancer autoimmune diseases, diabetes or allergic reactions.

## Description

### Field of the invention

The present invention is concerned with conjugates consisting of non-toxic peptidic T cell antigens and target cell binding partners and their use for therapy and for the manufacturing of therapeutic agents inducing specific cytotoxicity of T cells.

The use of this type of conjugates is not known as a strategy of immunotherapy and bridges the fields of cellular and humoral immunology. It uses binding partners of cell surface receptors which have the potential to transport peptides to target cells where they can be internalized, processed and re-expressed on the cell surface, rendering the cells vulnerable to a cell-mediated immune reaction.

### Background of the invention

Lanzavecchia et al. 1988 (1) described that patients injected with murine anti-tumor MAbs for cancer treatment possessed CD4 T cell clones specific for processed IgG. They postulated that some of the tumor remissions observed in patients treated with murine MAbs might be due to a CD4 T cell response directed against murine IgG peptides expressed on tumor cells. This finding was confirmed by the group of Courtnay-Luck and Epenetos (Kosmas et al. 1991) (2).

Chestnut and Grey (1983) (3) were the first to show in the mouse system that polyclonal rabbit anti-mouse Ig can be presented by B cells to T cells much more efficiently than normal rabbit IgG. Furthermore, Benjamin et al. (1986) (4) showed in a murine system that intravenously injected rat MAbs directed against murine cell surface antigens induced more anti-rat Ig antibodies than rat MAbs directed against soluble murine proteins. However none of these groups, who have contributed most to the study of antibodies as antigens, has suggested coupling antigenic peptides to antibodies in order to increase the antigenicity of tumor cells.

The fact that T lymphocytes, unlike B lymphocytes, recognize peptides derived from processed antigens, expressed on the cell surface with autologous MHC, has proved to be one of the major discoveries of the last decade in the field of immunology. Current thinking on antigen processing (Germain et al. 1986) (5); (Bevan, 1987) (6) is that exogenous antigens are internalized by antigen presenting cells (macrophage, B lymphocytes or denditric cells), processed within the endosomal/lysosomal compartment and expressed on the cell surface as a complex with MHC class II molecules. This complex can stimulate CD4 T cells, which are predominantly but not exclusively of the helper phenotype (Unanue and Allen 1987) (7); (Corradin and Lanzavecchia 1991) (8).

In contrast, antigens which are synthesized inside the cells, such as viral proteins or autologous proteins, are processed within the cytosol and transported into the endoplasmic reticulum by specific pumps called TAP.1 and TAP.2 where they form complexes with MHC class I molecules. Cell-surface expression of these MHC class I-peptide complexes can then activate CD8 cytotoxic T lymphocytes (CTL) (Towsend and Bodmer 1989) (9) ; (Bjorkman et al. 1987) (10).

It has also been shown that a peptide derived from an exogenously presented hepatitis B envelope antigen can be presented by MHC class I molecules on B cells to CD8 T lymphocytes (Yewdell et al. 1988) (11) ; (Barnaba et al. 1990) (12). More recently, Donnelly et al. 1993 (13) also showed that recombinant fusion proteins, consisting of a modified Pseudomonas exotoxin and peptides from the influenza virus, could be processed and presented with class I MHC molecules, despite the fact that these proteins were internalized into the target cells. The activation of CD8 cells by this method was attributed to the presence of the translocating domain in the modified Pseudomonas exotoxin, which directed the peptides to the cytoplasm. In addition, Kovacsovics-Bankowski et al. (1993) (14) have shown that chicken egg ovalbumin adsorbed to beads could be phagocytosed by macrophages then processed and presented on MHC class I molecules.

Concerning the presentation of endogenous antigen, several workers have shown that peptides from internally produced proteins can be processed and presented via the MHC class II pathway. This has been shown for instance by Weiss and Bogen 1991 (15) for the endogenously synthesised Ig light chain, which can be processed and expressed on the cell surface with MHC class II molecules. In their study, this elicited a TH1 and TH2 response which protected against the development of a B cell lymphoma.

Pichler's group has made an important contribution to the analysis of the processing of peptides coupled to antibodies directed against the T cell surface molecules CD2 or CD4 (Wyss-Coray et al. 1992) (16). They used these conjugates to demonstrate that activated T lymphocytes were able to efficiently process and express peptides bound to class II MHC. However they did not report any cytotoxicity and did not mention at all the use of such conjugates for tumor targeting or therapy.

Among the numerous previously described anti-tumor antibodies, those directed against the idiotype of each individual B cell lymphoma seem to be the most specific. A large series of B cell lymphoma patients has been injected with unconjugated, tailor made anti-idiotype MAbs, but only a few complete tumor remissions were observed (Miller et al. 1982) (17). In most cases there was no response to this treatment (Meeker et al. 1985) (18) although the use of interferon-γ slightly improved the percentage of responding patients (Brown et al. 1989) (19).

### Description of the invention

In one aspect, the invention discloses conjugates (fusion proteins) comprising a non-toxic, peptidic T cell antigen, which is capable of forming a complex with MHC class I or II molecules, and a binding partner for a surface receptor on mammalian target cells, whereby
a) the binding partner has a high degree of selectivity for said target cell,
b) the conjugate comprising the binding partner and the T cell antigen is internalized in said cell after binding of the binding partner to its surface receptor on said cells,
c) the T cell antigen is processed from the conjugate and expressed on the cell surface as a complex with MHC molecules, whereby said complex is recognized by the T cell receptor, thereby inducing cytotoxicity of T cells for the aforesaid target cells,
and their use for therapeutic purposes, especially for the treatment of cancer and for the manufacturing of a therapeutic agent.

The conjugates can be produced according to the state of the art, e.g. by chemical linking or by genetic engineering and expression of a DNA which encodes the conjugate (also named fusion protein).

In another aspect, there is provided a pharmaceutical composition comprising an effective amount of a conjugate according to the invention. The composition may further include an effective amount of immunostimulating factors, such as interleukin (IL), preferably IL 12, which will induce the proliferation of cytotoxic T lymphocytes.

The therapeutic compositions of the invention may be employed preferably in treating human patients suffering from cancer.

The peptide-antibody conjugate is not toxic like an immunotoxin. Thus it could be injected in relatively large amounts. The T cell antigen part will become immunogenic only when expressed on the surface of the target cells in association with MHC.

When expressed on the target cells, the immunogenic peptide should induce an authentic cellular immune response involving CD4 or CD8 lymphocytes which will lead to interleukin and/or TNF secretion with inflammation at the target cells, e.g. at the tumor site, and killing of the target cells expressing the foreign peptide.

It is therefore induced a specific cytotoxity of T cells for the mammalian target cells. Usually, these target cells are, in the normal state, not, or not efficiently enough, recognised by cytotoxic T cells and, therefore, not lysed sufficiently.

This T cell immune response directed against target cells, e.g. the tumor cells, expressing the foreign peptide, may help to recruit other T cell clones against autologous authentic tumor specific peptide like, for instance, MAGE-1 in melanoma (Traversari et al. 1992 (84)).

One of the definite advantages of using T cell antigens in the form of selected immunogenic peptides coupled to antibodies to target them to, e.g., tumor cells is that the patient can be pre-sensitized against such peptides by vaccination with either the virus or the tetanus toxoid and he will not have antibodies against the peptide (as has been shown for the P2 peptide of tetanus toxin, Valmori et al. 1992 (20)). Such antibodies might interfere with the tumor targeting of the conjugates.

The peptides will be recognized by the T lymphocytes only when after internalization and processing they become expressed on target cells in the context of MHC.

Further aspects and advantages of the present invention will be apparent upon consideration of the following detailed description of the invention, including the examples.

It is preferred to use fusion proteins with immunogenic peptides restricted by MHC class I, HLA, A1, A2.1, A3.2, A11 and A24 or mixtures thereof. With these five molecules one could cover the HLA phenotypes of more than 90 % of the Caucasian and Asian populations (Roche Milane Symposium (21)).

The functional determinants of HLA-A2.1 peptide interactions have been investigated in detail by A. Sette (21). The importance of hydrophobic anchor residues in positions 1, 2, 9 and 10 was demonstrated.

Also for the other of the five common immunogenic peptides restricted for MHC class I, peptide binding motives are known:

| | Amino acid position | |
|---|---|---|
| HLA molecule | 2 | 9, 10 |
| A1 | T,S | Y |
| A2.1 | L,M | V |
| A3.2 | V,L,M | K |
| A11.2 | T,V | K |
| A24 | Y | F,L |
| (T,S, etc is the single-letter code for amino acids.) | | |

Peptides expressed in connection with HLA-A2 and other HLA phenotypes are described by H.G. Rammensee (21). Peptides relevant in the context of HLA-A2.2, HLA-A3 and HLA-A11 are described in U. Utz et al. 1992 (88), K. Takahashi et al. 1991 (89) and R. Gavioli 192 (90).

A further preferred embodiment of the invention is a conjugate in which an anti-idiotype antibody against antibodies occurring on malignant B-lymphomas is used as a binding partner.

A further preferred embodiment of the invention are conjugates containing the T cell antigen in such bound form that the T cell antigen is capable of being split off, processed in the target cell and presented as a complex with MHC.

In a preferred embodiment of the invention, the binding between the T cell antigen and the binding partner is effected by a disulfide bridge, an ester bond capable of being split by esterase, a peptide linker capable of being split by protease and/or also by a non-covalent binding. As non-covalent bindings, for example, immunological bindings or such binding as via biotin/avidin or streptavidin, respectively, are preferred.

A conjugate of this kind consists of, for example, a bispecific antibody the specificity of which is directed against a cell surface receptor on tumor cells and the other specificity of which is directed against the T cell antigen or part thereof. Also, it is possible to couple the T cell antigen to another substance against which, in turn, the specificity of the bispecific antibody will be directed. For instance, the T cell antigen may contain further peptidic sequences which are recognized by the bispecific antibody. Another possibility resides in, for example, coupling the binding partner to streptavidin whilst the T cell antigen is coupled to biotin, and vice versa. Here it must be borne in mind, however, that the binding between the T cell antigen and biotin in the cell can be split in such a way that the T cell antigen can be presented in the context of the MHC complex.

The advantage of such non-covalently bound conjugates comprising a binding partner and a T cell antigen lies in the fact that these conjugates can be produced in a simple manner and, if required, individually for the respective patients. Thus it is possible, without difficulty, to prepare a large number of individual patient-specific conjugates from a plurality of individual components of T cell antigen and binding partners.

By an immunogenic, peptidic T cell antigen there is usually to be understood a peptide which is recognized by the T cell receptor in the context of MHC class I or MHC class II complex. Such antigens usually have a length of 12 to 25 amino acids (for recognition in the MHC II complex) or of 8 to 12 amino acids (for recognition in the MHC I complex). The T cell antigen may either be bound covalently or non-covalently to the binding partner in this form, or may be contained in a compound which, after internalization of the conjugate, releases the antigen in the cell in such a way that it can be presented, in the MHC complex, at the cell surface of the tumor cell. Such peptides are described in general in A. Sette 1993 (85).

The T cell antigens (synonym T cell epitopes) are substantially non-toxic in the absence of T lymphocytes. Substantially non-toxic means that these antigens have a considerably lower or preferably not detectable toxicity, compared to toxins such as Pseudomonas exotoxin. Examples of preferred T cell antigens are the tetanus peptides P2, P4, P30, influenza matrix peptides, plasmodium peptides. Preferred examples are, among others, influenza nucleoproteins (NP) 147-154, human immunodeficiency virus-1 CHN-1) gp 160 315-327 or murine cytomegalovirus (MCMV) pp 89 168-176, as described, for example, in Taylor et al. (22), Takahashi et al. (23), and Reddehase, M., et al. (24).

Alternatively, the T cell antigen could be a chemically modified peptide if it can be specifically recognized by the receptor of T lymphocytes. Such chemical modification may include, for instance, the addition of a metal such as nickel, since it has been shown that in certain allergic patients there are T cells which recognize a peptide with a bound nickel atom (Romagnoli et al. 1991 (25)). The peptide T cell antigen can also be modified by an organic molecule which enhances the immunogenicity (Romero et al. 1993 (26)). Furthermore, one may consider as T cell antigen a partially artificial organic molecule as long as it can be expressed in the context of MHC and can be used for sensitization of the patient's T cells to foreignize the tumor cells.

To enable presentation of a T cell antigen in the MHC class I complex on the surface of a tumor cell, it is preferred to use a conjugate which contains, in addition, a translocating domain. Such translocating domains are known to the skilled artisan and are obtained, for instance, from bacterial toxins, such as Pseudomonas exotoxin (J. Hwang et al. 1987 (87); Deres et al. 1989 (91)) or Diphtheria toxin.

Such toxins are substances which enrich on the surface of cells and act in the cytoplasma. The internalization of the toxin is thereby mediated by its translocating domain.

Most individuals in industrialized societies have been vaccinated against toxins such as tetanus toxoid and have memory T-cells specific for peptides derived, e.g., from one of the peptides P2, P4 or P30 from the tetanus toxin (Panina-Bordignon et al. 1989 (27). Therefore, simply by taking a sample of blood from normal individuals and isolating their PBMC, we can determine for each individual which peptide stimulates a specific T-cell response. This can be done using the individual's PBMC as antigen presenting cells and the standard assays for cell proliferation. This test will be essential in a clinical situation for determining the appropriate conjugate (containing peptides P2, P4 (for MHC I) or P30) to use in therapy. This method is, of course, useful for other conjugates according to the invention.

The complex comprising the T cell antigen and MHC class I or class II is recognized in vivo by the T cell receptor, expressed in association with the CD3 complex at the surface of CD8 T cells for MHC class I restricted peptides or CD4 T cells for MHC class II restricted peptides. As a result of the presentation of the immunogenic foreign T cell antigen on the surface of the tumor cells, they are recognized as foreign and become the target of an attack by T lymphocytes which have the natural function of eliminating foreign cells. In order to increase the number of T cells sensitized to the said T cell antigen the tumor patient can be vaccinated and boosted with a vaccine from which the peptide is a derivative of fragment, e.g. with the tetanus toxin before injection with the conjugate containing the relevant T cell antigen.

In addition to this the conjugate contains a binding partner for a surface receptor or marker, which is highly expressed on tumor cells and has a high degree of selectivity for tumor cells. Another feature of this binding partner is that it is internalized in the tumor cell after being bound to its surface receptor.

The receptor for the binding partner is a surface molecule on the target cells (e.g. tumor cells), which is expressed in considerably higher amounts and to a large extent on the target cells than on normal cells. Examples for suitable binding partners are antibodies, cytokines, melanoma stimulating hormone (MSH), other hormones and growth factors.

In the case of lymphomas it is preferred to use anti-idiotype monoclonal antibodies or their active fragments as binding partners, which are tumor-specific and patient-specific. To that end, a malignant lymphoma cell is taken from the patient, and anti-idiotype antibodies directed against this are prepared and employed in the conjugate. Such "tailormade" antibodies are already known. Since it has meanwhile been found that such antibodies confer a binding to the tumor cells not only for the patient they have been obtained from but also for a large number of other patients, a plurality of such antibodies has meanwhile become commercially available.

In the course of clinical trials the concept of shared idiotype emerged. It was observed that an anti-idiotype MAb prepared against one individual lymphoma could crossreact with a high degree of selectivity with an idiotype from another B cell lymphoma patient. Thus, by screening a panel of anti-idiotype MAbs on B lymphoma cells from a patient, it was often possible to identify an already existing hybridoma producing a Mab with a specificity for the idiotype of his B cell lymphoma (Parker et al. 1990 (28)).

Further B lymphocyte specific MAbs which are useful binding partners are the pan-B MAbs, such as anti-CD20, CD21, CD22 or anti-CD37. These MAbs, labeled with high doses of ¹³¹I, have been used with some success for B cell lymphoma therapy (Press et al. 1989 (29); Goldenberg et al. 1991 (30); Kaminski et al. 1993 (31); Press et al. 1993 (32)).

For the treatment of T cell lymphomas, MAbs specific for either the idiotype or the α or β chain of the T cell receptor (TCR) are useful in the conjugates according to the invention. Some of these anti-α or β chain MAbs from TCR were first described as anti-T cell idiotypes (Reinherz et al. 1983 (33); Carrel et al. 1986 (34)) but were subsequently found to be shared by 1 to 10% of normal T lymphocytes.

MAbs directed against the IL-2 receptor or the IL-4 receptor (Waldmann et al. 1988 (35); Larche et al. 1988 (36)) could be used in the antibody-peptide therapy according to the invention for the elimination of a lymphoid subpopulation in cases of auto-immune disease or allergy.

It is possible for any person skilled in the art to select, according to the state of the art, further suitable T cell antigens. This is described, for example, in A. Sette 1993 (84), which is part of the disclosure of this application.

Of the solid tumors, melanomas are known to express, in addition to class I, varying amounts of class II MHC, which can be enhanced by interferon-γ. A large panel of MAbs directed against melanoma associated antigens has been described (Carrel et al. 1991 (37)) and some of them, such as MAb HMB45 (Kapur et al. 1990 (71)), have been reported to be readily internalized after contact with the cell surface antigen (International Consensus Conference on Human Melanoma Antigens, Lausanne 1993 (39)).

Glioblastoma cells also show various degrees of expression of class II MHC which can be enhanced by interferon-γ. A certain percentage (40 to 60%) of glioma express a high density of the EGF receptor, which could be a good target for the conjugates (Kalofonos et al. 1989 (40); Humphrey et al. 1990 (41)), especially if the conjugates are injected locally in cystic tumors or after surgical removal of the major part of the tumor. In the case of an intracraneal injection of the conjugates it is preferred to inject also locally autologous peripheral blood lymphocytes containing effector T cells capapble of recognizing the tumor cells with immunogenic peptides on their surface associated with MHC.

Carcinoma cells rarely express class II MHC. Thus, it is preferred to use the class I processing pathway in order to apply a therapy to these tumors. Several MAbs with different degrees of selectivity for carcinoma cells have been described. Some of them, such as those reacting with the Erb-B2 receptor (Harwerth et al. 1992 (42); Batra et al. 1992 (43); Kasprzyk et al. 1992 (44); Wels et al. 1992 (45)) and/or the EGF receptor (Mendelsohn et al. 1992 (46)) or MAb A33 from the group of Old (Welt et al. 1990 (47)), are readily internalized by carcinoma cells and could be used in the method according to the invention.

In addition to the elimination of cancer cells, another possible application of the invention consists in the use of peptide-antibody conjugates or peptide-cytokine conjugates to eliminate or decrease the number of a subpopulation of normal cells, such as T lymphocytes in autoimmune diseases or B lymphocytes in allergic diseases.

Suitable T cell receptors against which monoclonal antibodies can be used as binding partners in the conjugates according to the invention for the treatment of autoimmune diseases are described in Urban et al. 1988 (78), Owhashi et al. 1988 (79), Acha-Orbea et al. 1988 (80), Acha-Orbea et al. 1989 (81), Burns et al. 1989 (82) and Bach et al. 1993 (83).

It is known that certain autoimmune diseases such as type I diabetes or multiple sclerosis are due in part to the presence of a limited number of activated T cell clones directed against autologous cells. One could prepare monoclonal antibodies directed against the T cell receptor α or β of the autoreactive T cell clones. These monoclonal antibodies coupled to immunogenic foreign peptides could be internalized and lead to the expression of the foreign peptide in the context of the MHC of the targeted T cell clones. Expression of these foreign peptides on the target T cell clones would lead to their elimination by other T lymphocytes recognizing the foreign peptides.

Alternatively, one could use Interleukin-2 coupled to foreign peptides which could be internalized by the subpopulation of activated T cells which express the IL-2 receptor. The injection of IL-2-foreign peptide conjugates would then lead to the elimination of part of the activated T cell population, by the remaining T lymphocytes recognising the foreign peptide.

Allergic diseases are known to be in part due to the secretion of IgE by B lymphocytes which switch from IgG to IgE antibody secretion under the influence of IL-4. One could use IL-4-foreign peptide conjugate in order to target the foreign peptide towards the IL-4 receptor expressing B lymphocytes. These B lymphocytes ready to switch to IgE production would be eliminated by T lymphocytes recognising the foreign peptides on their surface.

Alternatively, one could induce the elimination of B lymphocyte subpopulations which express IgE on their surface by injecting anti-IgE antibodies coupled to peptidic T cell antigens.

The further description will be divided into three parts (I, II, III), each of which concerns the development of a strategy of internalization of immunogenic MHC restricted peptides into target cells, as will be described in the examples. The internalization and expression of the peptide on the MHC induce the specific lysis of the target cells by cytotoxic lymphocytes. Part I deals with the internalization and expression of class II MHC restricted peptides. Part II deals with class I MHC restricted peptides. It describes different recombinant fusion proteins between antibody, peptide and the translocating domain of exotoxins which facilitate the processing of peptides through the class I pathway. Finally, part III describes fusion proteins of peptides and various binding proteins such as growth factors, cytokines, interleukins or hormones to direct peptides to target cell populations according to the method of the invention.

The peptide-MAb conjugates can also be made with F(ab')2 fragments instead of the intact MAb. The F(ab')2-peptide conjugates should have the advantage that it will not bind and be internalized by cells expressing Fc receptors. In addition, they have a shorter half-life and a better penetration into the tumor tissue than intact antibodies.

### I - Class II MHC restricted antibody-peptide conjugate

### a) Use of different class II MHC restricted peptides coupled to MAb specific for the Ig receptor of human B cell lymphomas

It is preferred to use a class II restricted peptide from tetanus toxin, such as P2 or P30 peptides. These peptides are well-characterized and act as "universal" helper epitopes since they can form immunogenic complexes with a wide range of class II molecules (Panina-Bordignon et al. 1989 (27); Demotz et al. 1989 (48)). As described for the peptide P2 in the examples, we first couple 4 to 5 molecules of a hetero-bifunctional crosslinker (SPDP) (Pharmacia Uppsala, Sweden) per molecule of an anti-µ chain MAb. The peptides, which are synthesized with N-terminal cystein, are then added and the conjugation reaction, which is directly proportional to the increase in optical density at 343 nm due to the release of free Pyridine-2-thione, is monitored spectrophotometrically.

The conjugates can be tested on B lymphoma and/or EBV-transformed cell lines whose MHC phenotype has been characterized. The conjugates are able to induce lysis of the target cells (in the ⁵¹Cr release assay) and proliferation of the T cell clones (using the ³H thymidine uptake assay). T cell clones specific for P2 or P30 peptides are available to persons skilled in the art and have been previously characterized (Demotz et al. 1989 (48)).

### b) Use of different MAbs against other internalizing structures expressed on human B cell lymphomas and normal B Lymphocytes as well as on other tumors expressing MHC class II

MAbs against pan B antigens such as CD20, CD21, CD22 and CD37, as well as MAbs against the IL-4 receptor, are coupled first to the P2 or P30 peptide and the conjugates are assessed for internalization and expression of the peptide with the class II MHC molecules of B cell lymphomas. The ⁵¹Cr release assay in presence of CD4 cytotoxic T cell clones are used to determine the efficiency of peptide internalization and expression.

In a preferred embodiment of the invention one can use this type of conjugate *in vivo* to eliminate or reduce the T cell or B cell populations involved in inflammatory or allergic reactions.

Activated T cells, which express MHC class II, can internalize, process and present peptides coupled to MAbs directed against the T cell receptor α or β chains. This kind of conjugate could be considered for immunotherapy of HTLV₁ leukemias, some of which are known to express class II MHC.

TCR (T cell receptor) specific MAb-peptide conjugates can also be used to attack a limited subset of activated T cells in auto-immune diseases such as multiple sclerosis, insulin dependent diabetes mellitus or myasthenia gravis. Another type of antibody-peptide conjugate could be made with an anti-IL-2 receptor MAb. If successful, it could be used either in the therapy of HTLV₁ leukemias, as originally proposed with unconjugated MAb against the IL-2 receptor by Waldmann et al. 1988 (35). It can also have a clinical application in decreasing the number of activated T cells in inflammatory diseases or in allograft rejection.

Further examples of MAb-peptide induced cytotoxicity can be shown with human tumor cell lines which constitutively express MHC class II or in which the class II expression can be induced by treatment with interferon-γ, such as melanoma (Carrel et al. 1985 (49)) or glioblastoma cell lines. MAb anti-MSH receptor or MAb HMB45 (Kapur et al. 1992 (38)), which have been shown to react with internalizing structures on melanoma cell lines, are preferred.

For glioblastomas, it is preferred to use an anti-EGF receptor MAb (e.g. EMD 55.900, from Merck (Darmstadt, Germany)) that has been used after radiolabelling with ¹²⁵I or ¹³¹I for glioma localization or therapy.

### c) Use of the murine BCL1 lymphoma and anti-Id MAb B₁ for targeting of peptides recognized by murine CD4 T cells.

For testing the *in vivo* therapeutic efficacy of the conjugates according to the invention a model using the well-characterized murine lymphoma BCL₁ (Slavin and Strober 1978 (50)) is useful. This lymphoma is of Balb/c origin and when injected back into Balb/c mice it produces a lethal lymphoma within 30 days. An anti-idiotype MAb has been produced which recognizes the BCL₁ Ig idiotype and indeed this MAb (B₁) has been used in a bi-specific Ab therapy regime (Brissink et al. 1991 (51)). A P30 peptide with two additional aspartate residues at the C and N termini is synthesized to make it more soluble in physiological buffers and will be used for SPDP coupling.

In addition to the hydrophilic residues, a tyrosine residue N-terminal to the cystein residue is preferred for the SPDP coupling reaction. The tyrosine is useful for directly labeling the peptide and monitor its stability and tumor localization *in vivo* (Mach et al. 1974 (52); Buchegger et al. 1989 (53); 1990 (54)).

For testing *in vivo* for BCL₁ lymphoma therapy, the MAb B₁-P30 conjugate is injected in Balb/c mice i.v. with BCL₁ and immunized with P30. The B₁-P30 conjugate is injected intravenously and the mice are monitored for tumor growth. The exact timing of the various injections is determined empirically, based on previous therapy regimens. Controls include injection of unconjugated MAb B₁ and injection of an irrelevant MAb coupled to peptide P30. The same model is used to test the rat anti-murine IL-4 receptor or the rat anti-murine pan B antigen, to show that the strategy described above in section b) could be used for treatment of B cell lymphomas.

The same system is useful for testing the other conjugates according to the invention.

### d) Clinical application of the MHC class II restricted antibody targeting.

Most individuals in industrialized societies have been vaccinated against tetanus toxoid and have memory T cells specific for peptides derived from one of the peptides P2 or P30 from the tetanus toxin (Panina-Bordignon et al. 1989 (27)). Therefore, simply by taking a sample of blood from normal individuals and isolating their PBMC, we can determine for each individual which peptide stimulates a specific T cell response. This can be done using the individual's PBMC as antigen presenting cells and the standard assays for cell proliferation. This test will be essential in a clinical situation for determining the appropriate conjugate (containing peptides P2 or P30) to use in therapy. This method is, of course, useful for other conjugates according to the invention.

It is preferred to establish EBV transformed cell lines from a series of normal individuals and test if anti-µ Mabs conjugated to tetanus toxoid peptides, selected as described above, can induce lysis either by MHC compatible cytotoxic CD4 cell lines or by the individuals' own T lymphocytes stimulated *in vitro* with the selected immunogenic peptide. These *in vitro* tests are useful to determine to what extent boosting with a tetanus toxoid vaccine will help in the induction of CD4 cytotoxic T lymphocytes.

In parallel, other MAbs against the IL-4 receptor and pan B antigens will be useful *in vitro* against the same EBV transformed target cells using autologous T lymphocytes stimulated both *in vitro* and *in vivo*.

The patient's peripheral blood lymphocytes will be tested for *in vitro* reactivity with the different MHC class II restricted peptides, both before and after boosting with the tetanus toxoid vaccine, in order to select the best peptide. The peripheral blood lymphocytes of the patient, after one or two *in vitro* stimulations with the selected peptide, will be tested for their capacity to lyse their own B lymphoma cells (if they have been cultivated) or their own EBV transformed B cells after incubation with a MAb-peptide conjugate. If the test is positive, the patient will receive a first injection of unconjugated anti-idiotype MAb, which should decrease the level of circulating idiotypic Igs, and a slowly increasing amount of the same anti-idiotype MAb coupled to the selected tetanus toxoid peptide. It should also be possible to expand the patients' P2 or P30-specific T cell clones *in vitro* and to re-inject them into the patient subsequent to the injection of the conjugate(s).

One problem will be the production of anti-idiotype MAbs for each patient. However, in the course of a radioimmunotherapy trial of B cell lymphomas performed at the San Diego Cancer Center with anti-idiotype MAbs labeled with ⁹⁰Y (Parker et al. 1990 (28)), it was shown that for a large percentage of patients, anti-idotype MAbs produced against idiotypes from other patients reacted with a high degree of specificity with their own lymphoma idiotype. This observation of shared idiotypes, greatly facilitated the use of anti-idiotype antibody therapy for each patient. At the present time, the IDEC Pharmaceutical Corporation in San Diego has established a panel of anti-idiotype antibodies prepared for a phase III clinical trial of the immunotherapy of non Hodgkins lymphoma, with the US Food and Drug Administration.

The patient will be monitored not only for a clinical response, but also to determine if the internalization of class II restricted peptides could help them to produce an autologous CD8 response against a processed autologous class I restricted peptide (Chakrabarti and Ghosh 1992 (55)).

### II - class I MHC restricted fusion proteins with exotoxin translocating domains and antibody-binding domains

### a) Influenza matrix peptide and Plasmodium bergei peptide internalization by human B cell lymphomas using anti-Ig MAbs or fusion proteins

The matrix peptide 57-68 is known to be immunogenic when presented to CD8 CTL as a complex with HLA-A2.1 (Gotch et al. 1987 (56)). We have used a modified M.P. 56-68 (which is perfectly soluble) (Bednarek et al. 1991 (57)) to make a conjugate with a MAb specific for the µ chain of human Ig.

The matrix peptide, coupled to an anti-µ chain MAb, can be internalized by B cells and presented on the MHC class I molecules, and an immunogenic peptide from Plasmodium bergei, coupled to an anti-idiotype MAb, can be internalized and presented, also via the MHC class I pathway, by the murine BCL₁ lymphoma model described by Slavin and Strober 1978 (50) and Brissinck et al. 1991 (58). In both cases, peptide specific CD8 CTL were able to selectively lyse the B lymphoma cells which had internalized and re-expressed the class I restricted peptides on their cell surface. However, the efficancy of the MHC class I peptide processing and expressing was lower than for MHC class II peptide conjugates.

A major goal of immunologists is to be able to stimulate a class I restricted CTL response against exogenous antigens. The problem is that in most cases when antigens are provided exogenously, they are not routed into the cytoplasm (from where they can be transported to the class I pathway). The method according to the invention provides a solution. This method is further improved by using translocation domains, e.g. of the bacterial toxin *Pseudomonas aeruginosa exotoxin* A (P.E) or a functional part of this domain.

A number of different therapeutic approaches have been developed using bacterial exotoxins. Some can be modified such that they no longer bind indiscriminately to every cell but are targeted to specific cell types. The toxins are targeted to the cell surface from where they are delivered to the endosomes. Fragments of some toxins, such as Pseudomonas exotoxin, are then translocated into the cytoplasm (Waldmann, 1992 (59); Donnelly et al. 1991 (13)) replaced the toxin domain of P.E with the sequences of class I restricted peptides and showed that target cells became sensitized for lysis by peptide specific MHC class I restricted CTL after exposure to the fusion proteins. However, this construct contained no cell-specific binding partner.

It is preferred to produce plasmids containing genes coding for a translocation domain and one of the class I restricted peptides already described, e.g. P.b. 249-260, or the influenza nucleoprotein peptide 147-154, both of which are restricted by H-2K^{d}, the human immunodeficiency virus 1 (HIV1) gp 160 derived peptide P18 (315-327), which is restricted by H-2D^{d}, and the murine cytomegalovirus (MCMV) pp 89 derived peptide 168-176, which is restricted by H-2L^{d}. A gene coding for a single chain antibody (ScFv) will be included in the construct to give the fusion proteins a target cell specificity. It is preferred to use a single chain derived from the anti-BCL₁ idiotype antibody B₁. After transfection into an appropriate strain of *E. coli* the fusion protein can be purified, e.g. by using a Ni column. The fusion protein is then tested, both *in vitro* and *in vivo*, on the BCL₁ target. This method targets class I restricted peptides to tumor cells and directs them to the cytosol.

### b) Internalization of the influenza matrix peptide in different target human cancer cells using fusion proteins with translocating domain and bindings partners for Erb-B2 and the EGF receptor and other internalizing structures.

The peptides can be conjugated with binding partners specific for other internalizing structures, which are important tumor markers, such as Erb-B2 (Wels et al. 1992 (45)), the EGF receptor (Kalofonos et al. 1989 (40)), the IL-4 receptor, which is associated with some cancers such as squamous cell carcinomas (Larche et al. 1988 (36); Puri et al. 1991 (60)), and the Tac chain of the IL-2 receptor which is present on various T cell lymphomas (Waldmann et al. 1988 (35)). For example, an anti-Erb-B2 MAb-matrix peptide conjugate is useful for targeting lysis by human CD8 CTL specific for the matrix peptide, to tumor cells expressing Erb-B2 receptor.

As with the class II peptides, another preferred approach is the use of fusion proteins with a binding partner directed against the T cell receptor. This strategy represents a very selective form of immunotherapy of T cell lymphomas and leukemias which express the TCR. It could also be used to eliminate autoreactive T cell clones in diseases such as rheumatoid arthritis, multiple sclerosis and insulin dependent diabetes mellitus.

One could use a number of peptides other than the influenza matrix peptide restricted to HLA-A2.1 restricted to different class I MHC allotypes.

### c) Use of the in vivo murine experimental model with the BCL1 lymphoma and the anti-idiotype MAb B1 coupled to different class I and class II restricted MAbs. Study of the requirement for CD4 T cells to provide held to CD8 T cells.

It is now well established that for CD8 cytolytic cells to be activated they need "help" from CD4 cells. One practical example of this is the co-injection of a class II peptide P30 together with the *Plasmodium bergei* 249-260 peptide, to stimulate CD4 cells to provide "help" for CD8 cells in Balb/c mice (Widmann et al. 1990 (61)). Using the BCL1 model, this helper effect can improve the therapeutic efficacy of the treatment. Another preferred approach is to make "mixed" conjugates of class II targeted and class I targeted peptides and test them, both *in vitro* and *in vivo*, on BCL₁.

### III - Use of interleukins, cytokines or hormones to introduce immunogenic peptides into target cells

Bacterial exotoxins have been used for a number of very promising therapeutic approach i.e. the use of cytokine-toxin conjugates. Using genetic engineering, recombinant proteins have been produced which contain for example IL-2 and a binding domain-deleted *Pseudomonas* exotoxin protein (Lorderboum-Galski et al. 1988 (62)). This immunotoxin was effective in experimental animal models (Kozak et al. 1990 (63)). Fusion proteins have also been produced with IL-4, IL-6, α-MSH, EGF and TNF-α (reviewed in Waldmann 1992 (35)). However, this approach has not yet been used with immunogenic peptides for *in vivo* targeting of an immune response to particular cell subpopulations.

Useful conjugates according to the invention are recombinant fusion proteins between cytokines, such as IL-2 or IL-4, and MHC class II restricted peptides. Such conjugates are targeted to, for example, adult T cell leukemia/lymphoma cells which express the high affinity IL-2 receptor whereas normal resting T-cells do not, or to T-cells expressing the IL-4 receptor. It has previously been shown that the MAb MR6, which binds to the human IL-4 receptor, could inhibit the IL-4 induced proliferation of cloned helper T cells and the production of IgE by polyclonal B cells (Larche et al. 1988 (36)).

To produce fusion proteins consisting of a cytokine, a class I restricted peptide and the translocation domain of *Pseudomonas extotoxin* A as described in section II. Once targeted to the cell surface the complex will be internalized to the endosomes from where the toxin translocation domain and the peptide will be targeted to the cytoplasm. The peptide can then be processed, transported to the E.R. and form a complex with MHC class I molecules. The targeting of a peptide to the class I processing pathway using the *Pseudomonas* exotoxin translocation domain has recently been demonstrated (Donnelly et al. 1991 (13)). However, there was no antibody or interleukin in this fusion protein to give them a target cell specificity, as described in the invention.

The advantage of the treatment according to the invention over the immunotoxin therapy is that the peptide of the conjugate according to the invention is non-toxic by itself. It will lead to the destruction of the target cells only when it is expressed in association with the MHC. The therapeutic efficacy of these fusion proteins can be tested *in vivo* using the BCL₁ model (transfecting BCL₁ with the genes coding for the IL-2 or IL-4 receptors).

### Comparison with other monoclonal antibody therapy approaches

In the bispecific antibody approach, MAbs containing two binding sites, one directed against the T cell receptor and the other against a tumor marker, can bridge T cells and tumor cells and direct the cytotoxicity of T cells to the tumor cells (Staerz et al. 1985 (64); Perez et al. 1985 (65)). The bispecific antibody approach has the advantage that it can use any activated T lymphocyte independently of its specificity. However, it represents only a passive form of therapy with no specific T cell antigen involved, whereas the method according to the invention truly bridges the fields of passive antibody therapy and the active induction of a T cell response in that it transports an active immunogen to the tumor cell. The active cellular antitumor response induced by the presence of foreign peptides on the tumor cells may, in turn, induce the recruitment of a broader T cell response against discrete autologous T cell antigens/peptidic tumor antigens such as those encoded by MAGE I described by the group of Boon in melanomas (van der Briggen et al. 1991 (66)). Another disadvantage of bispecific antibodies is that, once injected, they can immediately bind to circulating T cells in the peripheral blood and be eliminated, whereas with the method according to the invention the peptide-antibody conjugate will circulate freely and eventually reach the target cells.

The immunotoxin approach (Thorpe et al. 1988 (67); (Vitetta et al. 1990 (68)) is significantly different from the invention in that it does not involve T lymphocytes and the antibody-toxin conjugate has a relatively high degree of non-specific toxicity. In contrast, the conjugates according to the invention are not toxic, and they induce an active T cell immune response against the foreign peptides only when they will be expressed on the surface of target cells in the context of MHC.

Another very preliminary approach to tumor immunotherapy consisted in the transport of intact allogeneic MHC class I molecules to the tumor site with the help of antibodies (Savage and Epenetos 1992 (69)). This is clearly very different from using MAbs to transport immunogenic peptides to tumor cells.

### Description of the drawings

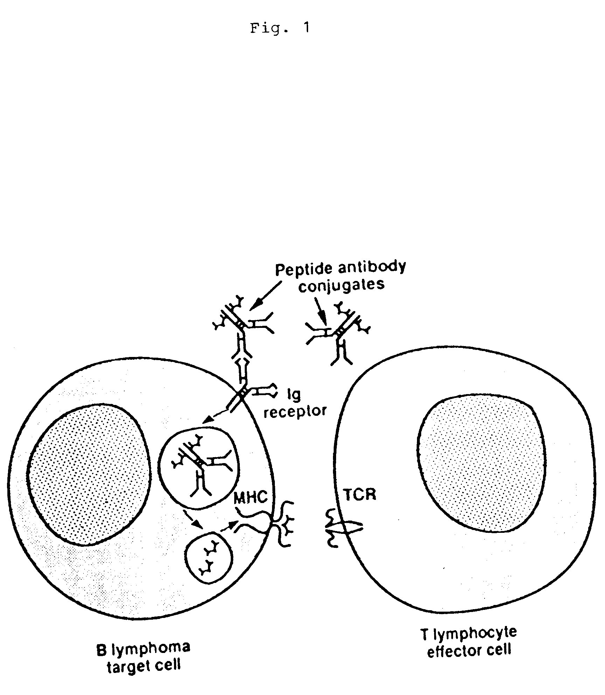

- Figure 1: The general concept of the peptide binding partner conjugate strategy is schematically illustrated. As an example, an anti-idiotype antibody peptide conjugate inducing the lysis of a B-lymphoma target cell is shown.

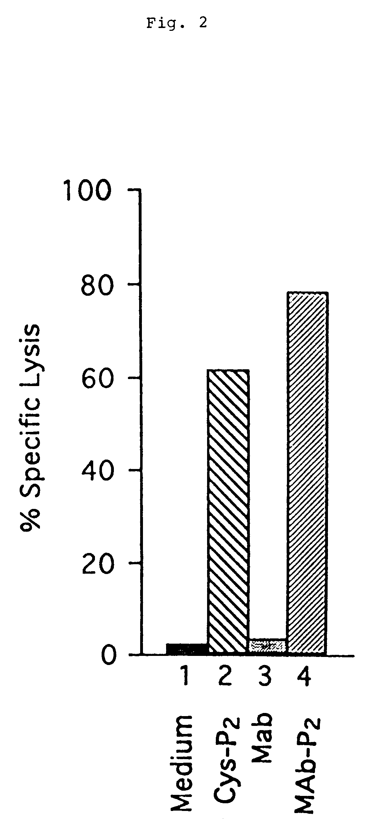

- Figure 2: Demonstration of the induction of T cells cytolysis of a B cell lymphoma by preincubation with tetanus toxin P2 peptide anti-λ MAb conjugate. The B-cell lymphoma Ramos target cells were labeled with ⁵¹Cr and plated in 100 µl of medium containing either medium alone (bar 1), free cystein-P2 peptide at 200 µM (bar 2), unconjugated anti-λ MAb at 1 µM (bar 3), or the P2 anti-λ MAb conjugate at a P2 concentration of 0.4 µM (bar 4) were added and the plates incubated for 60 1 hr at 37°C, at which time the SP2.2 clone was added at an effector to target ratio of 10:1, in 50 µl of medium. After 4 hr, 100 µl of supernatant was harvested and the ⁵¹Cr release measured. The tests were performed in duplicate and the results are representative of 3 experiments.

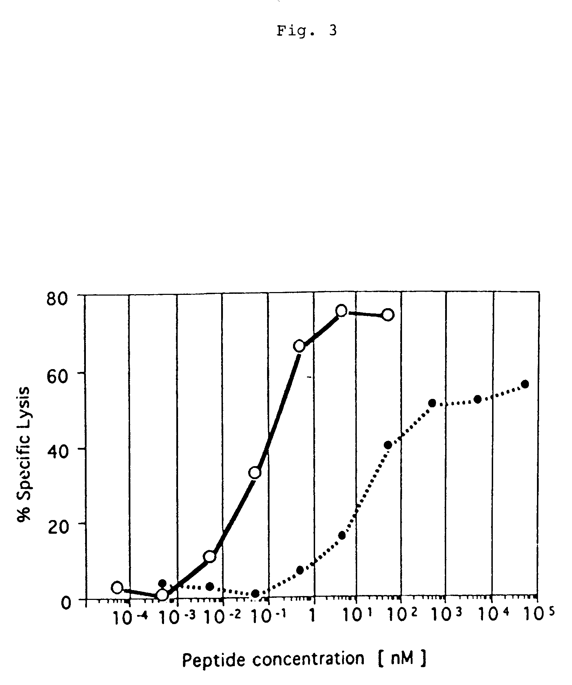

- Figure 3: Comparative titration of P2 anti-λ MAb conjugate and free cystein-peptide P2. The concentration of P2-anti-λ MAb refers to the calculated concentration of the peptide P2 coupled to the antibody. The ⁵¹Cr release test was performed as above with the indicated concentrations of free cystein P2 (black circles) and P2-anti-λ MAb (o--). The tests were performed in duplicate and the results are representative of three experiments.

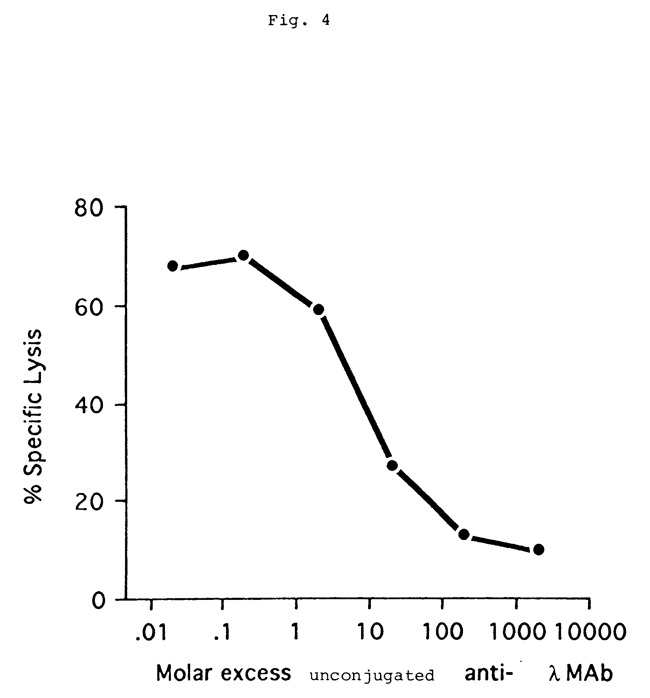

- Figure 4: Inhibition of the cytolysis induction by P2-anti-λ MAB conjugate by preincubation of the target cells with increasing amounts of unconjugated anti-λ MAb. ⁵¹Cr-labeled Ramos cells were pre-incubated for 30 min at 6000 cells per well in 100 µl of medium, together with the increasing concentrations of unconjugated anti-λ antibody expressed in molar excess of unconjugated MAb over P2-MAb indicated in abscissa. The P2-anti-λ conjugate was then added at 1.0 nM of coupled P2 peptide for a further 30 min, at which time SP2.2 clone was added at an effector to target ratio of 10:1. The tests were performed in duplicate and the results are representative of 2 experiments.

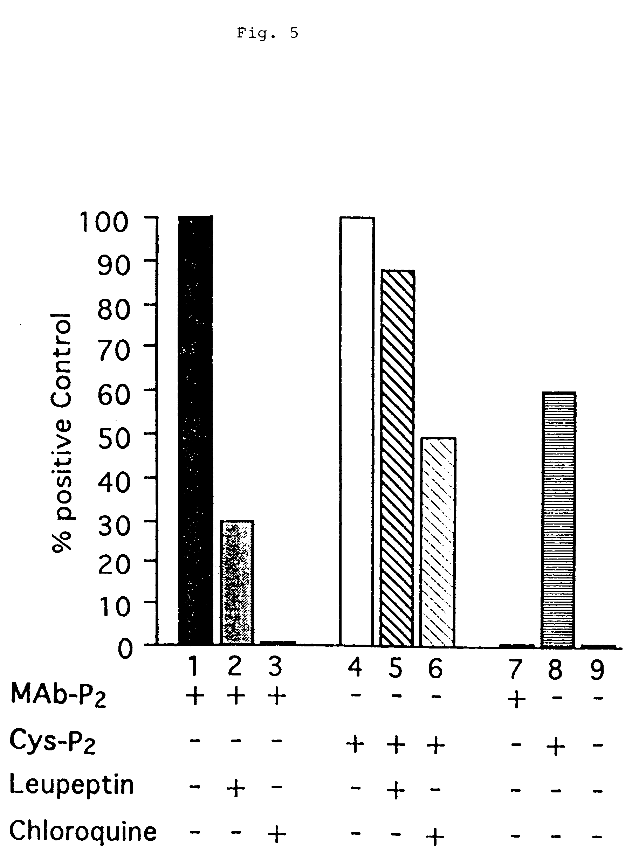

- Figure 5: T cell proliferation and inhibition by Leupeptin and Chloroquine. Ramos cells were pre-incubated for 3 hr at 37°C with either the P2-anti-λ conjugate at a P2 concentration of 0.2 µM (bars 1, 2, 3), 50 µM P2 peptide (bars 4, 5, 6) ± the inhibitors Chloroquine or Leupeptin at 300 µM and 400 µM respectively. The Ramos cells were then fixed with 0.025 % gluteraldehyde, washed three times, and plated at 30'000/well, with 20'000 SP2.2 cells. In addition, bars 7-9 show the proliferation results obtained with fixed Ramos cells (30'000/well) incubated with P2-MAb (at a P2 concentration of 0.2 µM) (bar 7), peptide P2 at 50 µM (bar 8) or medium alone (bar 9), for 30 min before adding the SP2.2 cells (20'000/well). After 24 hr at 37°C, 1 µCi of thymidine was added to all wells. Sixteen hours later the cells were harvested and the thymidine incorporation measured. The positive control level of proliferation with P2 was 14'000 CPM and with the P2-MAb 13'000. The background incorporation was less than 100 CPM. All tests were performed in triplicate; the standard deviations were less than 10 %. The results are representative of 3 experiments.

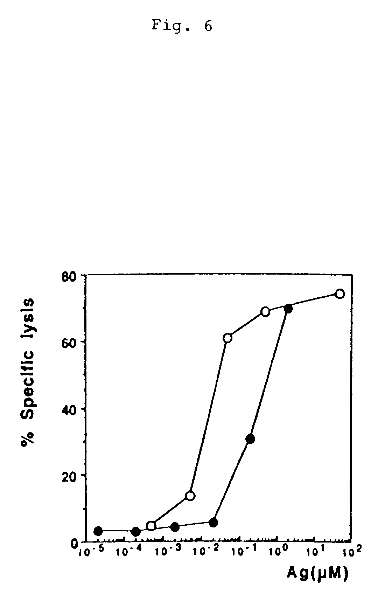

- Figure 6: The murine B lymphoma BCL₁ can be efficiently lysed by a Plasmodium bergei peptide specific CD8 T cell clone after incubation with a conjugate consisting of the peptide and the anti-BCL₁ idiotype MAb B₁ (o (black circles) ― o). The positive control consists of the free peptide (o ― o).

### Example 1

### The peptide P2 universal epitope from tetanus toxin coupled to anti-light chain MAb can induce lysis of human B lymphoma cells by specific CD4 T lymphocytes

Well characterized MHC restricted immunogenic peptides coupled to antibodies directed against a relevant tumor marker can be internalized into tumor cells, processed and expressed together with the class II MHC molecule. The consequence of peptide expression by tumor cells is that they could then be specifically attacked by T lymphocytes sensitized by vaccination to this peptide. In other words, we show that a tumor cell can be "foreignized" or rendered antigenic by an antibody mediated internalization of a selected MHC restricted immunogenic peptide. The strategy of the antibody-peptide conjugate approach to cancer immunotherapy is schematically represented in figure 1.

After a two hour incubation with a conjugate consisting of the tetanus toxin peptides P2 (830-843), P4 and P30 coupled to an anti-light chain Ig MAb (a substitute for an anti-idiotype MAb), the human B lymphoma cell line Ramos (ATCC CRL 1596) was efficiently lysed by a cytolytic CD4 T lymphocyte clone specific for the peptide P2.

Such T cell clones for in vitro testing of the therapeutic efficacy can be obtained by selecting T cell clones from natural individuals vaccinated with the toxin which is the basis for the peptide (in this example, tetanus toxoid). Selection criteria are cell proliferation and toxicity after stimulation with the related peptide (Demotz et al., 93 and Panina-Bordignon et al. (27)).

Antibody without peptide or anti-κ chain antibody with the coupled peptide did not induce any lysis. As shown in figure 2, incubation with free peptide also induced lysis of the B lymphoma target cells by T lymphocytes but at a molar concentration 500 to 1000 higher than that of the coupled peptide.

We also showed that the induction of specific cytolysis by the human anti-peptide CD4 T cell clone could be specifically inhibited with unconjugated anti-λ MAb. The results obtained with the cytotoxicity assay were confirmed by proliferation assays i.e. the peptide P2 specific T cell clone proliferated selectively in presence of Ramos cells pulsed with the peptide P2 anti-λ chain conjugate.

To further determine by which route the conjugate was being processed, we used the lysosomotropic agents Chloroquine and Leupeptin. Chloroquine is a weak base that can increase the pH of acidic vesicles thus interfering both with the enzymatic-activity and lysosomal mobility involved in the class II antigen processing pathway. Both Chloroquine and Leupeptin have also been shown to affect the dissociation of the invariant chain from the class II molecule in the endosomes, which would prevent peptide binding (Nowell et al. 1985 (70); Germain and Margulies 1993 (71)). The results clearly show that Chloroquine and Leupeptin can inhibit the conjugate induced proliferation of the P2 peptide specific T cell clone.

Overall, the results clearly demonstrate that anti-Ig MAbs represent very efficient vectors for the internalization and processing of immunogenic peptides in B lymphoma cells, which are subsequently rendered susceptible to lysis by cytotoxic CD4 lymphocyte clones raised against the relevant peptide. Therefore, an anti-idiotype antibody will be able to selectively transport this and other tetanus toxin peptides to B lymphoma cells, which will process the conjugates and present the peptides to the patient's CD4 T cell repertoire. Our tetanus toxoid vaccinated population has T cells specific for the immunogenic peptide and these, in principle, could be further expanded prior to injection of the conjugate.

The conjugate mediated induction of a CD4 T cell response directed to the B cell lymphoma expands CD8 T cell precursors specific for peptides derived from the Ig molecule and presented with the class I MHC. It has already been shown, in a different model system, that T cells are able to recognize processed autologous antibody-derived idiotypic peptides bound to MHC class I molecules (Chakrabarti and Ghosh 1992 (55)).

### Material and Methods

### Peptides

P2 was synthesized using an automated (Applied Biosystems 431 A, Foster City, USA) peptide synthesiser. Peptides were purified on a G25 Sephadex column and then by HPLC. A cysteine residue was added to the amino terminus of P2 maintained in reduced form by keeping it in 0.01 M acetic acid until used for coupling to MAb. The peptide was analyzed for amino acid composition. The sequence of Cys-P2 is: Cys-Gln-Tyr-Ile-Lys-Ala-Asn-Ser-Lys-Phe-Ile-Gly-Ile-Thr-Glu (Free Cys-P2 was used throughout the example).

### Antibodies

The mouse anti-human λ chain MAb of IgG2a isotype and the control antihuman κ chain also of IgG2a isotype were purified by ammonium sulfate precipitation and ion-exchange column from ascites obtained in Balb/c mice after injection of the ATCC cell lines, HP6054 and HP6053, respectively, (Reimer et al. 1984 (72)). It is also possible to use an anti-human µ chain MAb DA4-4 (ATCC HB57), or others.

### Antibody-Peptide Conjugates

The anti-λ and anti-κ MAb, were first derivatized using the heterobifunctional crosslinker SPDP (Succrinimidyl-3(2-pyridyldithio)propionate) (Pharmacia Upsalla, Sweden) at a SPDP/antibody molar ratio of 10:1 (according to manufacturer's protocol). Cys-P2 was then added to the derivatized antibody and the conjugation reaction was monitored spectrophotometrically by the increase of optical density at 343 nm due to the release of free Pyridine-2-thione. The release of Pyridine-2-thione measured at 343 nm is proportional to the amount of peptide coupled to the antibody.

We calculated that an average of 5 peptide molecules was coupled to the anti-λ MAb, using the formula:
Molar concentration of the peptide molecules conjugated:
ΔA343 nm/8.08 x 10³ M⁻¹ cm⁻¹ x 1 cm

### T-cell clones

The P2-peptide specific CD4 cytolytic T-cell clone SP2.2, known to be HLA-DR7 restricted and obtained as described (Panina-Bordignon et al. 1989 (27)). It was maintained in RPMI 1640 culture medium supplemented with 5 % human serum (Swiss Red Cross, Bern, Switzerland), 1 % sodium pyruvate, 1 % L-glutamine, 1 % non-essential amino acids, 40 µg/ml Kanamycin (Gibco, Paisley, Scotland) and 100 U/ml human recombinant IL-2 (Roche , Nutley, USA). The clone was restimulated every 15 - 20 days with 1 µg/ml of phytohaemagglutinin (Sigma, St. Louis MO), and allogeneic irradiated peripheral blood mononuclear cells (PBMC) as feeder cells, as previously described (Lanzavecchia, Nature 1985 (86)).

### Cell lines

The B-cell lymphoma Ramos was provided by Dr. S. Carrel. The Ramos cells were shown to express the Ig λ light chain and were confirmed to be homozygous for HLA-DR7 by oligonucleotide hybridization genotyping (Tiercy et al. 1991 (73)).

### The ⁵¹Cr release assay

The ⁵¹Cr release assay was performed as previously described (Brunner et al. 1968 (74); Healy et al. 1988 (75)). Briefly, 2 x 10³ (for the EBV transformed B cell line EKR) or 6 x 10³ (for lymphoma lines) of ⁵¹Cr labeled cells in 100 µl of medium were added to the individual wells of V-bottomed 96 well plates (Greiner, Nürtingen, Germany). Each well contained 50 µl of either peptide or antibody-peptide conjugate at various concentrations. T-cell clones, at a constant effector to target ratio of 10:1, were then added in 50 µl of medium. After 4 hr at 37°C the plates were centrifuged, 100 µl of supernatant were collected and the ⁵¹Cr release measured in a gamma counter. Competition experiments were performed as above using fixed concentrations of the antibody-peptide conjugates and a molar excess of unconjugated antibody, as indicated in the Results section.

### Proliferation and inhibition assays

These were performed using 20'000 T cells together with 30'000 Ramos B cells as antigen-presenting cells (APC) and 50 µM of Cys-P2 peptide or 0.2 µM of conjugated P2 peptide (in triplicate) in flat-bottomed 96-well plates in a final volume of 200 µl of culture medium. One µCi of [³H] thymidine was added at 24 hr and the incorporated thymidine measured after 40 hr of culture.

For the inhibition experiments, Ramos APC were preincubated with or without either Chloroquine at 300 µM (Sigma) or Leupeptin at 400 µM (Sigma) for 30 min and then, without washing, for a further 3 hr incubation with peptide or conjugate. After two washings, the cells were then fixed with 0.025 % glutaraldehyde, washed three times again, and the proliferation test carried out as previously described (Demotz et al. 1989 (48)).

### Results

The tetanus toxin peptide P2 with a cysteine residue at the N-terminal position was coupled to an anti-human λ light chain MAb and chromatographed on Sephadex G200. A peptide-antibody conjugate with an average molar ratio of 5, which was eluted from the Sephadex column just before a 7 S reference IgG, was selected for all subsequent experiments. The conjugate was tested in comparison with free peptide Cys-P2 for its capacity to induce the lysis of the B lymphoma cells Ramos by the cytolytic P2-specific CD4 T cell clone SP2.2. Figure 2 shows that a 0.1 µM concentration of peptide present on the conjugate gives a higher level of lysis than 50 µM of free peptide. The anti-human λ antibody alone induced no lysis.

We then titrated the free peptide and P2-antibody conjugate on Ramos cells to compare their respective capacity to induce cytolysis. As can be seen from figure 3, the P2 conjugate is around 500 to 1000 times more efficient at inducing lysis of Ramos cells than the free Cys-P2 peptide. For example, the concentration of coupled P2 peptide required for a 40 % lysis is more than 1000 fold lower than free Cys-P2. The plateau of maximum lysis is also higher (75 %) for the coupled peptide compared with free Cys-P2 (55 %). Similar results were obtained with free P2 peptide with and without cystein.

In order to determine how the peptide-antibody conjugates were being taken up and processed, we undertook a series of inhibition experiments. First, we showed that by using a fixed concentration of the P2-antibody conjugate and increasing concentrations of the unconjugated anti-λ antiody, we could obtain a specific inhibition of the conjugate induced lysis with a 30 fold molar excess of anti-λ antibody (Fig. 4). In addition, isotype matched control anti-κ antibody P2 conjugate did not induce Ramos cell lysis. This, together with the fact that neither of the two conjugates could induce lysis of the Ig negative DR7 positive target EKR, whereas the free peptide Cys-P2 shows that the conjugate is internalized and processed after binding to cell surface Ig. It also rules out that the induction of lysis is due to internalization of the conjugate via Fc receptor or its degradation on the cell surface.

To further determine by which route the conjugate was being processed, we used the lysosomotropic agents Chloroquine and Leupeptin. Chloroquine is a weak base that can increase the pH of acidic vesicles thus interfering both with the enzymatic-activity and lysosomal mobility involved in the overall class II antigen processing pathway. Both Chloroquine and Leupeptin have also been shown to affect the dissociation of the invarian chain from the class II molecule in the endosomes, which could prevent peptide binding (Germain and Margulies 1993 (76)). The results clearly show that Chloroquine and Leupeptin can inhibit the conjugate induced proliferation of the T-cell clone SP2.2 (Fig. 5, bars 2 and 3), whereas Leupeptin had almost no effect on free peptide induced proliferation (Fig. 5, bar 5). Chloroquine, however, did reduce the P2-induced proliferation by 50 % (Fig. 5, bar 6), but the conjugate induced proliferation was inhibited by 100 % (bar 3). Conuugates incubated with gluteraldehyde fixed APC could not induce proliferation, confirming the requirement for internalization (bar 7), whereas Cys-P2 peptide incubated with fixed APC was sufficient to induce proliferation (bar 8) indicating that the DR7 molecule on the fixed APC cell surface was functionally intact. Glutaraldehyde fixation did not alter the antigenicity of the λ chain since the anti-λ chain antibody gave a very positive binding to fixed cells as determined by FACS.

### Example 2

### Class I restricted peptides coupled to an anti-Ig MAb can be internalized and presented by the MHC class I molecules of B lymphoma cells

### a)

First, we coupled, according to example 1, a modified form of the influenza matrix peptide 57-68 to an anti-µ chain MAb DA 4-4 (ATCC HB57). Since the original peptide described by Gotch et al. 1987 (56) is not soluble, we replaced the amino terminal amino acids by hydrophilic amino acids, as first described by Bednarek et al. 1991 (57); this peptide is completely soluble. We have used a matrix peptide specific T cell line, HC12, which is obtained as previously described (Healy et al. 1988 (75) and McMichael et al. 1977 (77)).

The peptide was modified by substituting a lysine for a glycine at position 58, an alanine for an isoleucine at position 59, an aspartic acid for a threonine at position 67, and a lysine for a valine at position 68.

Influenza virus specific CTL lines are prepared as described in McMichael et al. 1977 (77) and in Healy et al. 1988 (75). Briefly, fresh peripheral blood mononuclear cells (PBMC) were stimulated with autologous irradiated PBMC previously exposed to influenza virus (10 Hemaglutinin units/10⁶ cells) in RPMI 160 medium, in the absence of serum, for 90 min. at 37°C (stimulator cells). Responder cells (2 x 10⁶/ml) in normal medium with 10 % human AB serum for a period of 10 days. The responding T cells were then restimulated under the same conditions for a further 7 days and thereafter continuously growing IL-2 dependent lines were maintained by weekly restimulations of 5 x 10⁵ responder T cells and 2 x 10⁶ stimulator cells in medium containing IL-2.

Using this T cell line and a HLA-A2.1 B cell lymphoma target cell line, pre-incubated for 6o min at 37° with the peptide anti-µ chain conjugate at a peptide concentration of 0.2 micromolar, we obtained 40% specific lysis in the ⁵¹Cr release assay with the conjugate compared to 60% lysis with a 20 times higher concentration of free peptide ; the negative control was 3%.

### b)

A further system which we have tested employed the murine B cell lymphoma BCL₁ (H2K^{d})^{.}This cell line was incubated for 2 hrs at 37°C with a conjugate consisting of a MHC class I restricted peptide from *Plasmodium bergei* (amino acids 249 - 260) coupled to the anti-BCL₁ idiotype MAb B1 according to example 1. The CD8 clone was derived from Balb/c mice immunized with the same peptide (Slavin and Strober 1978 (50)). In this system we obtained 68% specific lysis with the conjugate, which is comparable with the 66% obtained with a 5 times lower molar concentration of free peptide; the negative control lysis was 5% (Fig. 6).

These data show that class I restricted peptides can be presented by B lymphocytes when exogenously introduced in the form of an anti-Ig-peptide conjugate.

However, the molarity of class I MHC restricted peptides on the conjugate to obtain 50 % of target cell lysis (0.5 µmol/l) is much higher than that necessary to obtain the same lysis with conjugates containing class II restricted peptides (0.1 nmol/1). That is why a translocation domain will be preferably useful to direct the expression of class I restricted peptides.

All references are herein incorporated by reference. Although the present invention has been described in some detail by way of illustration and example for purposes of clarity and understanding, it will be apparent that certain changes and modifications may be practiced within the scope of the appended claims.

The mentioned cell lines are only examples of cell lines useful in the examples. It is apparent that other cell lines bearing the same or essentially the same properties are also useful for illustrating the invention.

### REFERENCES

1. Lanzavecchia, A., Abrignani, S., Scheidegger, D., Obrist, R., Dorken, B., and Moldenhauer, G. Antibodies as antigens. The use of mouse monoclonal antibodies to focus human T cells against selected targets. J Exp Med, *167*: 345-352, 1988.
2. Kosmas, C., Epenetos, AA., and Courtenay-Luck, NS. Patients receiving murine monoclonal antibody therapy for maligancy develop T cells that proliferate *in vitro* in response to these antibodies as antigens. Br J Cancer, *64*: 494-500, 1991.
3. Chestnut, R.W. and Grey, H.M. Studies on the capacity of B cells to serve as antigen presenting cells. J Immunol, *126*: 1075-1082, 1981.
4. Benjamin, R.J., Cobbold, S.P., Clark, M.R., and Waldmann, H. Tolerance to rat monoclonal antibodies. Implications for serotherapy. J Exp Med, *163*: 1539-1552, 1986.
5. Germain, R.N. The ins and outs of antigen processing and presentation. Nature, *322*: 687, 1986.
6. Bevan, M.J. Class discrimination in the world of immunology. Nature, *325*: 192-194, 1987.
7. Unanue, E.R. and Allen, P.M. The basis for the immunoregulatory role of macrophages and other accessory cells. Science, *236*: 551-557, 1987.
8. Corradin, G. and Lanzavecchia, A. Chemical and functional analysis of MHC class II-restricted T cell epitopes. Int Rev Immunol, *7*: 139-147, 1991.
9. Towsend, A.R. and Bodmer, H. Antigen recognition by class I-restricted T lymphocytes. Ann Rev Immunol, *7*: 601-624, 1989.
10. Bjorkman, P., Saper, M.A., Samroui, B., Bennet, W.S., Strominger, J.L., and Wiley, D.C. Structure of the human class I histocompatibility antigen, HLA-A2. Nature, *329*: 506-512, 1987.
11. Yewdell, J.W., Benninck, J.R., and Hosaka, Y. Cells process exogenous proteins for recognition by cytotoxic T lymphocytes. Science, *239*: 637-640, 1988.
12. Barnaba, V., Franco, A., Alberti, A., Benvenuto, R., and Balsano, F. Selective killing of hepatitis B envelope antigen-specific B cells by class I-restricted, exogenous antigen-specific T lymphocytes. Nature, *345*: 258, 1990.
13. Donnelly, J.J., Ulmer, J.B., Hawe, L.A., Friedman, A., Shi, X.-P., Leander, K.R., Shiver, J.W., Oliff, A.I., Martinez, D., Montgomery, D., and Liu, M.A. Targeted delivery of peptide epitopes to class 1 major histocompatibility molecules by a modified *Pseudomonas* exotoxin. Proc Natl Acad Sci USA, *90*: 3530-3534, 1993.
14. Kovacsovics-Bankowski, M., Clark, K., Benacerraf, B., and Rock, K.L. Efficient major histocompability complex class I presentation of exogenous antigen upon phagocytosis by macrophages. Proc Natl Acad Sci USA, *90*: 4942-4946, 1993.
15. Weiss, S. and Bogen, B. MHC class II-restricted presentation of intracellular antigen. Cell, *64*: 767-776, 1991.
16. Wyss-Coray, T., Brander, C., Bettens, F., Mijic, D., and Pichler, W.J. Use of antibody/peptide constructs to direct antigenic peptides to T cells : evidence for T cell processing and presentation. Cellular Immunol, *139*: 268-273, 1992.
17. Miller, R.A., Maloney, D.G., Warnke, R., and Levy, R. Treatment of B-cell lymphoma with monoclonal anti-idiotype antibody. N Engl J Med, *306*: 517-522, 1982.
18. Meeker, T.C., Lowder, J., Maloney, D.G., Miller, R.A., Thielemans, K., Warnke, R., and Levy, R. A clinical trial of anti-idiotype therapy for B cell malignancy. Blood, *65*: 1349-1363, 1985.
19. Brown, S., Miller, R.A., Horning, S.J., Czerwinski, D., Hart, S.M., McElderry, R., Basham, T., Warnke, R.A., Merigan, T.C., and Levy, R. Treatment of B-cell lymphomas with anti-idiotype antibodies alone and in combination with alpha interferon. Blood, *73*: 651-661, 1989.
20. Valmori et al., J. Immunol. 149 (1992) 717 - 721.
21. Roche Milane Ricerche Opening Symposium, Milane, Italy, Sept. 9/11, 1993, Conference contribution by A. Sette; Abstracts of A. Sette and J.F. Bach.
22. Taylor et al., Immunogenetics 26 (1987) 267.
23. Takahashi et al., Proc. Natl. Acad. Sci. USA 85 (1988) 3105.
24. Reddehase M. et al., Nature 337 (1987) 651.
25. Romagnoli et al., Embo J. 10 (1991) 1303 - 1306.
26. Romero et al., J. Immunol. 150 (1993) 3825 - 3831.
27. Panina-Bordignon, P., Tan, A., Termijtelen, A., Demotz, S., Corradin, G., and Lanzavecchia, A. Universally immunogenic T cell epitopes : promiscuous binding to human MHC class II and promiscuous recognition by T cells. Eur J Immunol, *19*: 2237-2242, 1989.
28. Parker, B.A., Vassos, A.B., Halpern, S.E., Miller, R.A., Hupf, H., Amox, D.G., Simoni, J.L., Starr, R.J., Green, M.R., and Royston, I. Radioimmunotherapy of human B-cell lymphoma with ⁹⁰Y conjugated antiidiotype monoclonal antibody. Cancer Res, *50 (Suppl.)*: 1022s-1028s, 1990.
29. Press, O.W., Eary, J.F., Badger, C.C., Martin, P.J., Appelbaum, F.R., Levy, R., Miller, A., Brown, S., Nelp, W.B., Krohn, K.A., Fisher, D., DeSantes, K., Porter, B., Kidd, P., Thomas, E.D., and Bernstein, I.D. Treatment of refractory non-Hodgkin's lymphoma with radiolabeled MB-1 (Anti-CD37) antibody. J Clin Onc, *7*: 1027-1038, 1989.
30. Goldenberg et al., J. Clin. Oncology 9 (1991) 548 - 568.
31. Kaminski et al., New England Journal of Medicine 329 (1993) 459 - 465.
32. Press et al., New England Journal of Medicine 329 (1993) 1219 - 1224.
33. Reinherz, E.L., Meuer, S.C., and Schlossman, S.F. The human T cell receptor: analysis with cytotoxic T cell clones. Immunol Rev, *74*: 83-112, 1983.
34. Carrel, S., Isler, P., Schreyer, M., Vacca, A., Salvi, S., Giuffre, L., and Mach, J.-P. Expression on human thymocytes of the idiotypic structures (Ti) from two leukemia T cell lines Jurkat and HPB-ALL. Eur.J Immunol., *16*: 649-652, 1986.
35. Waldmann, T.A., Goldman, C.K., Bongiovanni, K.F., Sharrow, S.O., Davey, M., Cease, K.B., Greenberg, S.J., and Longo, D.L. Therapy of patients with human t-cell lymphotrophic virus i- induced adult t-cell leukemia with anti-tac, a monoclonal antibody to the receptor for interleukin-2. Blood, *72*: 1805-1816, 1988.
36. Larche, M., Lamb, J.R., O'Hehir, R.E., Imami-Shita, N., Zanders, E.D., Quint, D.E., Moqbel, R., and Ritter, M.A. Functional evidence for a monoclonal antibody that binds to the human IL-4 receptor. Immunol, *65*: 617-622, 1988.
37. Carrel, S., Doré, J.-F., Ruiter, D.J., Prade, M., Lejeune, F.J., Kleeberg, U.R., Rümke, Ph., and Bröcker, E.B. The EORTC melanoma group exchange program: evaluation ofa multicenter monoclonal antibody study. Int J Cancer, *48*: 836-847, 1991.
38. Kapur, R.P., Bigler, S.A., Skelly, M., and Gown, A.M. Anti-melanoma monoclonal antibody HMB45 identifies an oncofetal glycoconjugate associated with immature melanosomes. J Histochem.Cytochem., *40*: 207-212, 1992.
39. International Consensus Conference on Human Melanoma Antigens, Lausanne 1993, Abstracts.
40. Kalofonos, H.P., Pawlikowska, T.R., Hemingway, A., Courtenay-Luck, N., Dhokia, B., Snook, D., Sivolapenko, G.B., Hooker, G.R., McKenzie, C.G., Lavender, P.J., Thomas, D.G.T., and Epenetos, A.A. Antibody Guided Diagnosis and Therapy of Brain Gliomas using Radiolabeled Monoclonal Antibodies Against Epidermal Growth Factor Receptor and Placental Alkaline Phosphatase. J Nucl Med, *30*: 1636-1645, 1989.
41. Humphrey, P.A., Wong, A.J., Vogelstein, B., Zalutsky, M.R., Fuller, G., Archer, G.E., Friedman, H.S., Kwatra, M.M., Bigner, S.H., and Bigner, D.D. Anti-synthetic peptide antibody reacting at the fusion junction of deletion-mutant epidermal growth factor receptors in human glioblastoma. Proc Natl Acad Sci U S A, *87*: 4207-4211, 1990.
42. Harwerth, I.-M., Wels, W., Marte, B.M., and Hynes, N.E. Monoclonal antibodies against the extracellular domain of the erbB-2 receptor function as partial ligand agonists. J Biol Chem, *21*: 15160-15167, 1992.
43. Batra, J.K., Kasprzyk, P.G., Bird, R.E., Pastan, I., and Richter King, C. Recombinant anti-erbB2 immunotoxins containing *Pseudomonas* exotoxin. Proc Natl Acad Sci USA, *89*: 5867-5871, 1992.
44. Kasprzyk, P.G., Uk Song, S., Paolo Di Fiore, P., and Richter King, C. Therapy of an animal model of human gastric cancer using a combination of anti-erbB2 monoclonal antibodies. Cancer Res, *52*: 2771-2776, 1992.
45. Wels, W., Harwerth, I.-M., Mueller, M., Groner, B., and Hynes, N.E. Selective inhibition of tumor cell growth by a recombinant single-chain antibody-toxin specific for the erbB-2 receptor. Cancer Res, *52*: 6310-6317, 1992.
46. Atlas, I., Mendelsohn, J., Baselga, J., Fair, W.-R., Masui, H., and Kumar, R. Growth regulation of human renal carcinoma cells : role of transforming growth factor alpha. Cancer Res, *52*: 3335-3339, 1992.
47. Welt, S., Divgi, C.R., Real, F.X., Yeh, S.D., Garin Chesa, P., Finstad, C., Sakamoto, J., Cohen, A., Sigurdson, E.R., Kemeny, N., and et al., Quantitative analysis of antibody localization in human metastatic colon cancer: a phase i study of monoclonal antibody a33. J Clin Oncol, *8*: 1894-1906, 1990.
48. Demotz, S., Lanzavecchia, A., Eisel, U., Niemann, H., Widmann, C., and Corradin, G. Delineation of several DR-restricted tetanus toxin T cell epitopes. J Immunol., *142*: 394-402, 1989.
49. Carrel, S., Schmidt-Kessen, A., and Giuffrè, L. Recombinant interferon-gamma can induce the expression of HLA-DR and -DC on DR-negative melanoma cells and enhance the expression o HLA-ABC and tumor-associated antigens. Eur J Immunol, *15*: 118, 1985.
50. Slavin, S. and Strober, S. Spontaneous murine B-cell leukemia. Nature, *272*: 624, 1978.
51. Brissink, J., Demanet, C., Moser, M., Leo, O., and Thielemans, K. Treatment of mice bearing BCL1 lymphoma with bispecific antibodies. J Immunol, *147*: 4019-4026, 1991.
52. Mach, J.P., Carrel, S., Merenda, C., Sordat, B., and Cerottini, J.C. In vivo localisation of radiolabelled antibodies to carcinoembryonic antigen in human colon carcinoma grafted into nude mice. Nature, *248*: 704-706, 1974.
53. Buchegger, F., Pfister, C., Fournier, K., Prevel, F., Schreyer, M., Carrel, S., and Mach, J.P. Ablation of human colon carcinoma in nude mice by 131I-labeled monoclonal anti-carcinoembryonic antigen antibody F(ab')2 fragments. J Clin.Invest, *83*: 1449-1456, 1989.
54. Buchegger, F., Pèlegrin, A., Delaloye, B., Bischof-Delaloye, A., and Mach, J.-P. 131-I labeled F(ab')2 fragments are more efficient and less toxic than intact anti-CEA antibodies in radioimmunotherapy of large human colon carcinoma grafted in nude mice. J Nucl Med, *31*: 1035-1044, 1990.
55. Chakrabarti, D. and Ghosh, S.K. Induction of syngeneic cytotoxic T lymphocytes against a B cell tumor. Cellular Immunol, *144*: 455-464, 1992.
56. Gotch, F., Rothbard, J., Howland, K., Towsend, A., and McMichael, A. Cytotoxic T lymphocytes recognize a fragment of influenza virus matrix protein in association with HLA-A2. Nature, *326*: 881, 1987.
57. Bednarek, M.A., Engl, S.A., Gammon, M.C., Lindquist, J.A., Porter, G., Williamson, A.R., and Zweerink, H.J. Soluble HLA-A2.1 restricted peptides that are recognized by influenza virus specific cytotoxic T lymphocytes. J Immunol Methods, *139*: 41-47, 1991.
58. Brissinck, J., Demanet, C., Moser, M., Leo, O., and Thielemans, K. Treatment of mice bearing BCL₁ lymphoma with bispecific antibodies. J Immunol, *147*: 4019-4026, 1991.
59. Waldmann, T.A. Immune receptors : Targets for therapy of leukemia/lymphoma, auto-immune diseases and for the prevention of allograft rejection. Annu Rev Immunol, *10*: 675-704, 1992.
60. Puri, R.K., Ogata, M., Leland, P., Feldman, G.M., FitzGerald, D., and Pastan, I. Expression of high-affinity interleukin 4 receptors on murine sarcoma cells and receptor-mediated cytotoxicity of tumor cells to chimeric protein between interleukin 4 and *Pseudomonas* exotoxin. Cancer Res, *51*: 3011-3017, 1991.
61. Widmann, C., Romero, P., Maryanski, J.L., Corradin, G., and Valmori, D. T helper epitopes enhance the cytotoxic response of mice immunized with MHC class I-restricted malaria peptides. J Immunol Methods, *155*: 95-99, 1992.
62. Lorderboum-Galski, H., Kozak, R., Walmann, T., Bailon, P., FitzGerald, D., and Pastan, I. IL-2-PE40 is cytotoxic to cells displaying either the p55 or p75 subunit of the IL-2 receptor. J Biol Chem, *263*: 18650-18656, 1988.
63. Kozak, R.W., Lorderboum-Galski, H.J., Willingham, M.C., Malek, T., FitzGerald, D.J.P., Waldmann, T.A., and Pastan, I. IL-2-PE40 prevents the development of tumors in mice injected with IL-2 receptor expressing EL4 transfectant tumor cells. J Immunol, *145*: 2766-2771, 1990.
64. Staerz, U.D., Kanagawa, O., and Bevan, M.J. Hybrid antibodies can target sites for attack by T cells. Nature, *314*: 628-631, 1985.
65. Perez, P., Hoffman, R.W., Shaw, S., Bluestone, J.A., and Segal, D.M. Specific targeting of cytotoxic T cells by anti-T3 linked to anti- target cell antibody. Nature, *316*: 354-356, 1985.
66. Van der Briggen et al., Science (1991).
67. Thorpe, P.E., Wallace, P.M., Knowles, P.P., Relf, M.G., Brown, A.N., Watson, G.., J., , Blakey, D.C., and Newell, D.R. Improved antitumor effects of immunotoxins prepared with deglycosylated ricin a-chain and hindered disulfide linkages. Cancer Res, *48*: 6396-6403, 1988.
68. Vitetta, E.S. and Yen, N. Expression and functional properties of genetically engineered ricin b chain lacking galactose-binding activity. Biochim Biophys Acta, *1049*: 151-157, 1990.
69. Savage, P. and Epenetos, A.A. Two step immunotargeting in cancer. Imperial Cancer Research Fund, Scientific Report, *101a22*: 436, 1992. (Abstract)
70. Nowell, J. and Quaranta, V. Chloroquine affects biosynthesis of Ia molecules by inhibiting dissociation of invariant (g) chains from a-b dimers in B cells. J Exp Med, *162*: 1371-1376, 1985.
71. Germain, R.N. and Margulies, D.H. The biochemistry and cell biology of antigen processing and presentation. Annu Rev Immunol, *11*: 403-450, 1993.
72. Reimer et al., Hybridoma 3 (1984) 263 - 275.
73. Tiercy et al., Proc. Natl. Acad. Sci. USA 88 (1991) 7121 - 7125.
74. Brunner et al., Immol. 14 (1968) 181 - 188.
75. Healy, F., Sire, J., Gomard, E., Yssel, H., Jordan, BR., and Lévy, J.-P. A study of functionally active amino acids involved in the interaction of HLA-A2 or HLA-A3 molecules with cytolytic T lymphocytes. J Immunol, *141*: 2487, 1988.
76. Germain and Margulies, Ann. Rev. Immol. 11 (1993) 403 - 450.
77. McMichael et al., Nature 270 (1977) 324.
78. Urban, J.-L. et al. (1988). Restricted use of T cell receptor V genes in murine autoimmune encephalomyelitis raises possibilities for antibody therapy. Cell, 54: 577 - 592.
79. Owhashi, M. et al. (1988). Protection from experimental allergic encephalomyelitis conferred by a monoclonal antibody directed against shared idiotype on rat T cell receptors specific for myelin basic protein. J. Exp. Med., 168: 2153.
80. Acha-Orbea, H. et al. (1988). Limited heterogeneity of T cell receptors from lymphocytes mediating autoimmune encephalomyelitis allows specific immune intervention. Cell, 54: 263 - 273.
81. Acha-Orbea, H. et al. (1989). T cell receptors in murine autoimmune diseases. Ann. Rev. Immunol., 7: 371 - 405.
82. Burns, F.R. et al. (1989). Both rat and mouse T cell receptors specific for the encephalitogenic determinant of myelin basic protein use similar V a and V b chain genes even though the major histocompatibility complex and encephalitogenic determinants being recognized are different. J. Exp. Med., 169: 27 - 39.
83. Bach, J.-F., Abstract: Milan Symposium (21), Immunotherapy of type I diabetes (1993).
84. C. Traversari et al., J. Exp. Med. 176 (1992) 1453 - 1457.
85. A. Sette ed., Naturally processed peptides, Chem. Immol. 1993, Vol. 57, published by Karger, Basle.
86. Lanzavecchia et al., Nature 314 (1985) 537 - 539.
87. J. Hwang et al., Cell 48 (1987) 128 - 136.
88. U. Utz et al., J. Immunol. 149 (1992) 214 - 221.
89. K. Takahashi et al., Proc. Natl. Acad. Sci. USA 88 (1991) 10277 - 10281.
90. R. Gavioli et al., Proc. Natl. Acad. Sci. USA 89 (1992) 5862 - 5866.
91. Deres et al., Nature 342 (1989) 561 - 564.

## Claims

1. Conjugate comprising an essentially non-toxic peptidic T cell antigen, which is capable of forming a complex with MHC class I or II molecules, and a binding partner for a surface receptor on mammalian target cells, whereby
a) the binding partner has a high degree of selectivity for said target cells by binding to receptors which are expressed on the surface of said target cells,
b) the conjugate comprising the binding partner and the T cell antigen is internalized in said cells after binding of the binding partner to its surface receptor on said target cells,
c) the T cell antigen is processed from the conjugate and expressed on the cell surface in the form of a complex with MHC molecules, whereby said complex is recognized by the T cell receptor, thereby inducing cytotoxicity of T cells for the aforesaid target cells.

2. The conjugate as claimed in claim 1, characterized by additionally containing a translocating domain which will favor the presention of the T cell antigen in the form of a complex with MHC class I molecules on the surface of the aforesaid target cells.

3. The conjugate as claimed in claims 1 to 2, characterized in that, as a binding partner, an anti-idiotype antibody against surface immunoglobulin receptors expressed on malignant B lymphomas is used or an anti-idiotype antibody reacting with a restricted α or β chain from the T cell receptor on T lymphomas.

4. The conjugate as claimed in claim 1 or 2, characterized in that as an binding partner, a growth factor, cytokine, an interleukin or a hormone for which the said target cell has a receptor, is used.

5. The conjugate as claimed in the preceding claims, characterized in that the T cell antigen and the binding partner are bound in such form that the binding is split by substances endogenously contained in the target cell.

6. The conjugate as claimed in claim 5, characterized in that the binding is a disulfide bridge, an ester bond capable of being split by esterase, or a peptide bond capable of being split by protease.

7. Use of a conjugate as claimed in one of the preceding claims to induce specific cytotoxicity of T cells for mammalian target cells.

8. Use of a conjugate as claimed in one of claims 1 to 6 for the elimination of a subpopulation of non-malignant B or T lymphocytes, for the treatment of autoimmune diseases, diabetes or allergic reactions.

9. Use of a conjugate as claimed in claims 1 to 6 in tumor therapy.
